# EUROPEAN PATENT APPLICATION

(11) **EP 1 987 811 A1**
(43) Date of publication of application: **05.11.2008**
(21) Application number: 07713841.0
(22) Date of filing: 06.02.2007
(51) Int. Cl.: A61K 8/49, A61K 31/381, A61P 17/16, A61P 43/00, A61Q 19/02, C07D 333/22

(54) **SKIN-WHITENING COSMETIC**

(30) Priority: 14.02.2006 JP 2006036842; 31.10.2006 JP 2006296552
(71) Applicant: Pola Chemical Industries Inc., Shizuoka-shi, Shizuoka 422-8009 (JP)
(72) Inventor: KATAGIRI, Takayuki, Kanagawa 244-0812 (JP); YOKOYAMA, Kouji, Kanagawa 244-0812 (JP); KIMURA, Makoto, Kanagawa 221-0833 (JP); SAITOH, Yuko, Kanagawa 244-0812 (JP)
(74) Representative: Bannerman, David Gardner
(86) International application number: PCT/JP2007/051999
(87) International publication number: WO 2007/094200

(57) **Abstract**

Disclosed are: a novel skin-whitening agent; and a skin-whitening cosmetic or quasi-drug which is highly effective for prevention/amelioration of melanopathy and is prepared using the skin-whitening agent. The cosmetic contains a compound represented by the general formula (I) and /or a salt thereof as an active ingredient. In the general formula (I), R¹ and R² independently represent a secondary or tertiary alkyl group having 3 to 7 carbon atoms; and R³ represents a hydrogen atom, an alkyl group having 1 to 4 carbon atoms or an alkoxy group having 1 to 4 carbon atoms.

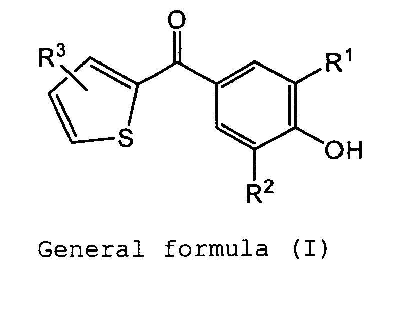

## Description

### [Technical Field]

The present invention relates to a skin-whitening cosmetic. The term "cosmetic" used in the present invention also includes an equivalent to a product classified as a quasi-drug in Japan.

### [Background Art]

A spot, freckle, and pigmentation after skin is exposed to the sun refer to a condition in which melanogenesis is sharply accelerated by activation of pigment cells (melanocyte) which exist in the skin. There has been known a skin external preparation(especially skin-whitening agent) to prevent or ameliorate such skin pigment troubles containing ascorbic acids, hydrogen peroxide, colloidal sulfur, glutathione, hydroquinone, or catechol (see e.g., Non-patent Document 1). However, it is also known that such whitening agents sometimes effectively exert their actions but sometimes do not exert the actions. Under the present circumstances, the causes have not been elucidated in detail.

Moreover, the above-described skin-whitening agents sometimes cause stability and safety problems. That is, ascorbic acid, which is used for preventing or ameliorating pigmentation, may be easily oxidized and unstable in a system containing water at a high concentration, such as a high water content cosmetic, and it may change the color of a skin preparation for external use (skin-whitening agent). Meanwhile, a hydrogen peroxide solution has problems of its preservation stability and safety, while glutathione and colloidal sulfur have very unusual odors and are difficult to use as components of skin-whitening agents. In addition, hydroquinone, catechol, etc. may have safety problems such as dermal irritation and allergic property. In view of such technological background, a novel skin-whitening material that can effectively act and has satisfactory stability and safety has been desired.

On the other hand, a thiophene derivative, represented by the general formula (I) below, is known to have an antioxidant effect, anti-inflammatory effect provided through a mechanism caused by suppression of prostaglandin, anti-allergic effect, and antirheumatic effect (see, Patent Documents 1, 2, 3, and 4 and Non-Patent Documents 2 and 3, for example).

However, it has not been known that such a compound has a melanin formation inhibitory action in the skin, and a skin-whitening cosmetic containing the compound as an active ingredient has not been known at all.

[Patent Document 1] JP 53-141265 A
[Patent Document 2] JP 63-008380 A
[Patent Document 3] JP 63-502281 A
[Patent Document 4] JP 06-501919 A
[Non-patent Document 1] "Usability of cosmetics, Evaluation Techniques and future perspectives", edition by Katsuyuki TAKEDA et al., edited by YAKUJINIPPO LIMITED. (2001)
[Non-patent Document 2] Agents and Action, Vol. 12, No. 5, p. 674-683, (1982)
[Non-patent Document 3] Bioorganic & Medicinal Chemistry, Vol. 11, p4207-4216, (2003)

### [Disclosure of the Invention]

The present invention has been made in view of the above-described circumstance, and an object of the present invention is to provide a cosmetic that is highly effective for prevention/amelioration of melanopathy such as spots and freckles.

The inventors of the present invention have made extensive studies to achieve the above-described objects, and, as a result, the inventors have found out that a specific thiophene derivative (specifically, a compound represented by the general formula (I) and/or a salt thereof) has a strong effect of inhibiting melanin formation in melanocytes. The inventors have further found out that a base material such as cosmetic containing the thiophene derivative has an excellent effect of preventing or ameliorating melanopathy on the skin, thus completing the present invention. That is, the present invention is as follows.

(1) A skin-whitening cosmetic containing a compound represented by the following general formula (I) and/or a salt thereof.

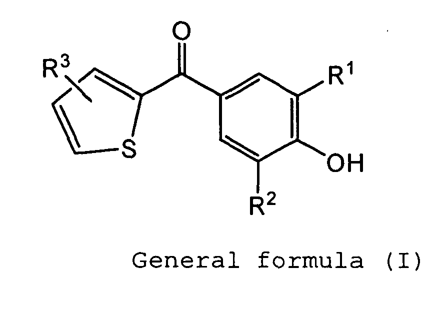

(In the general formula (I), R¹ and R² independently represent a secondary or tertiary alkyl group having 3 to 7 carbon atoms, and R³ represents a hydrogen atom, an alkyl group having 1 to 4 carbon atoms, or an alkoxy group having 1 to 4 carbon atoms.)
(2) A skin-whitening cosmetic according to the item (1), in which the compound represented by the general formula (I) is a compound represented by the following general formula (II).

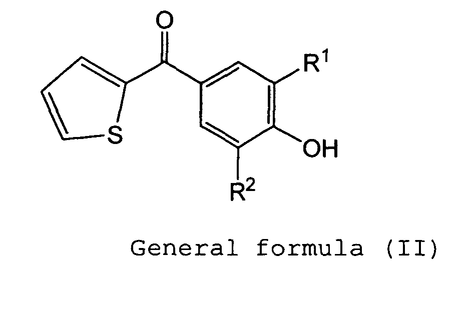

(In the general formula (II), R¹ and R² independently represent a secondary or tertiary alkyl group having 3 to 7 carbon atoms.)
(3) A skin-whitening cosmetic according to the item (2), in which the compound represented by the general formula (II) is 2,6-di-tert-butyl-4-(2'-thenoyl)phenol (Compound (1)).

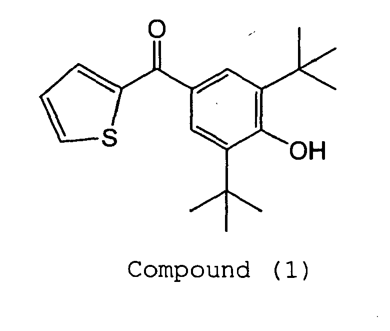

(4) A skin-whitening cosmetic according to any one of the items (1) to (3), which is used for prevention/amelioration of melanopathy.
(5) A skin-whitening cosmetic according to any one of the items (1) to (4), in which a content of the compound represented by the general formula (I) is 0.0001 to 3% by mass.
(6) A skin-whitening cosmetic according to any one of the items (1) to (5), further containing at least one polyhydric alcohol selected from 1,3-butanediol, dipropylene glycol, isoprene glycol, 1,2-pentanediol, 2,4-hexylene glycol, 1,2-hexanediol, and 1,2-octanediol.

The present invention also includes use of a compound represented by the general formula (I) and/or a salt thereof for production of a skin-whitening cosmetic and a method including applying a compound represented by the general formula (I) and/or a salt thereof to the skin to be whitened.
Preferable aspects of the compound represented by the general formula (I), preferable use of the cosmetic, and preferable components to be further incorporated in the above-described use and method of the present invention are the same as those described in the items (2) to (6) above.

### [Brief Description of the Drawing]

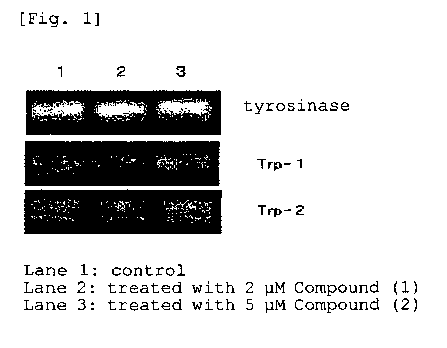

[Fig. 1] A drawing (substitute photograph for drawing) showing the results of Western blotting, which shows expression of tyrosinase, Trp-1, and Trp-2 proteins.

### [Best Mode for carrying out the Invention]

(1) Compound represented by general formula (I): essential component of cosmetic of the present invention
The compound that is an essential component of a cosmetic of the present invention is represented by the general formula (I). In the general formula (I), R¹ and R² independently represent a secondary or tertiary alkyl group having 3 to 7 carbon atoms, i.e., a bulky substituent. Specific examples of each of R¹ and R² include an isopropyl, isobutyl, sec-butyl, tert-butyl, isoamyl, sec-amyl, and tert-amyl group, respectively. Of those, the tert-butyl group is preferable, and a di-tert-butyl compound, where both of R¹ and R² are tert-butyl-groups, is more preferable.

In the general formula (I), R³ may be a hydrogen atom or any substituent selected from alkyl groups having 1 to 4 carbon atoms or alkoxy groups having 1 to 4 carbon atoms. In the case where R³ is a substituent, a substitution site of the substituent is not particularly limited. R³ is preferably an alkyl group having 1 to 4 carbon atoms or an alkoxy group having 1 to 4 carbon atoms. Of those, R³ is particularly preferably a hydrogen atom. That is, in the present invention, the compound represented by the general formula (I) is preferably a compound represented by the general formula (II).
The compound that is an essential component of a cosmetic of the present invention is further preferably 2,6-di-tert-butyl-4-(2'-thenoyl)phenol, i.e., a compound represented by the above-described chemical formula of Compound (1).

The compound represented by the general formula (I) may be used in a free form or as a salt obtained by using an alkali or the like for a cosmetic of the present invention. Examples thereof preferably include: alkali metal salts such as sodium and potassium salts; alkaline earth metal salts such as calcium and magnesium salts; organic amine salts such as ammonium, triethanolamine, and triethylamine salts; and basic amino acid salts such as lysine and arginine salts.

Those compounds can be produced by any method, and examples of the method include, but are not limited to, a method summarized as the following scheme 1 or 2. As will be understood by those skilled in the art, the followingmethodsmaybe appropriately modified to produce a target compound.

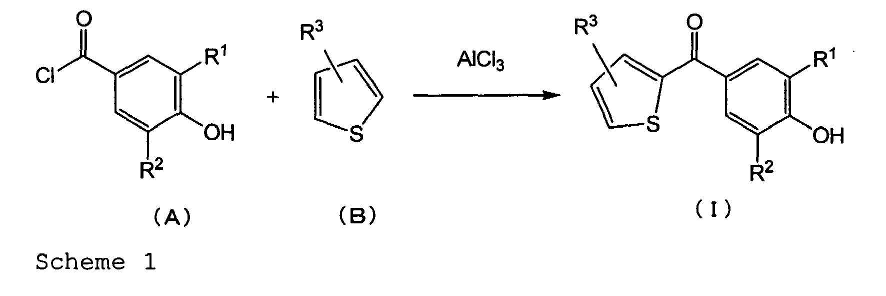

The reaction in scheme 1 will be described briefly. 3,5-di-alkyl-4-hydroxybenzoic acid is treated with thionyl chloride in accordance with a conventional method, to thereby produce an chloride of 3,5-di-alkyl-4-hydroxybenzoic acid, compound (A). The acid chloride compound (A) is treated with a Lewis acid such as aluminum chloride or titanium tetrachloride in an appropriate solvent such as carbon disulfide. Subsequently, thiophene, compound (B) is added to perform a Friedel-Crafts reaction, to thereby produce a compound represented by the general formula (I).

3,5-di-alkyl-4-hydroxybenzoic acid, for example, 3,5-di-tert-butyl-4-hydroxybenzoic acid commercially available from Sigma-Aldrich Corporation may be used. The compound (B) may be an unsubstituted compound where R³ is a hydrogen atom or may be a substituted compound such as methylthiophene or methoxythiophene. In the case where thiophene is used as the compound (B), a compound represented by the general formula (II) can be produced, while in the case where an acid chloride compound of 3,5-di-tert-butyl-4-hydroxybenzoic acid is used as the compound (A), 2,6-di-tert-butyl-4-(2'-thenoyl)phenol (Compound (1)) can be produced.

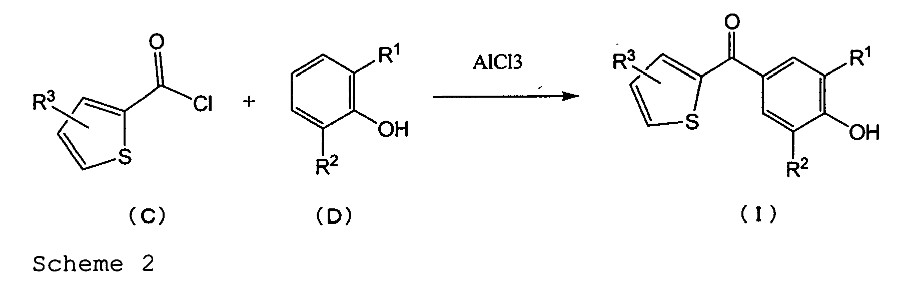

The reaction in scheme 2 will be described briefly. Thiophenecarboxylic acid is treated with thionyl chloride in accordance with a conventional method, to thereby produce an acid chloride of thiophenecarboxylic acid, compound (C). The acid chloride compound (C) is treated with a Lewis acid such as aluminum chloride or titanium tetrachloride in an appropriate solvent such as carbon disulfide. Subsequently, 2, 6-dialkylphenol (D) is added to perform a Friedel-Crafts reaction, to thereby produce a compound represented by the general formula (I).
In order to produce the compound (C), 2-thiophenecarboxylic acid, 3-methyl-2-thiophenecarboxylic acid, 5-methyl-2-thiophenecarboxylic acid, or the like, where R³ is a hydrogen atom, and which are commercially available from Sigma-Aldrich Corporation, may be used. In the case where an acid chloride of 2-thiophenecarboxylic acid is used as the compound (C), a compound represented by the general formula (II) can be produced. 2,6-dialkylphenol (D) may be 2,6-di-tert-butyl-phenol, 2,6-di-isopropylphenol, or the like, commercially available from Sigma-Aldrich Corporation. In the case where an acid chloride of 2-thiophenecarboxylic acid and 2,6-di-tert-butyl-phenol are used as the compounds (C) and (D), respectively, 2-6-di-tert-butyl-4-(2'-thenoyl)phenol (Compound (1)) can be produced.

A compound represented by the general formula (I) has an excellent melanin formation inhibitory action. In general, many of compounds having melanin formation inhibitory actions inhibit melanin formation through mechanisms of direct inhibitory action of tyrosinase and decomposition of tyrosinase. However, the compound represented by the general formula (I) is considered to inhibit melanin formation through a mechanism different from mechanisms of direct inhibitory action of tyrosinase and decomposition of tyrosinase.

### (2) Cosmetic of the present invention

A cosmetic of the present invention contains a compound represented by the general formula (I) and/or a salt thereof as an essential component. The cosmetic of the present invention may contain a compound represented by the general formula (I) or a salt thereof singly or in combination of two or more.

Although the content of the compound represented by the general formula (I) in a cosmetic of the present invention may vary depending on the purpose of the cosmetic, skin condition, dosage form, and application site, the content is 0.0001% by mass or more, preferably 0.001% by mass or more, and further preferably 0.01% by mass or more. Meanwhile, the upper limit is 3% by mass or less, preferably 1% by mass or less, and further preferably 0.8% by mass or less.
The compound represented by the general formula (I) of the present invention may be used in a quasi-drug in Japan or a similar product thereof in other countries because the compound has a strong melanin formation inhibitory action.

The cosmetic of the present invention is particularly preferably used at the skin site without inflammation. That is, the cosmetic of the present invention is preferably not used for anti-inflammation. In general, the cosmetic of the present invention is preferably applied to prevention of melanopathy such as spots and freckles on the skin, where melanin formation is enhanced but no inflammatory reaction or the like is caused, or preferably applied to a site with melanopathy.

The cosmetic of the present invention has a melanin formation inhibitory action and has an effect of preventing/ameliorating melanopathy such as spots and freckles. Therefore, in a preferable embodiment of the present invention, an indication of the effect/effectiveness of the cosmetic, for example, "this cosmetic can suppress melanin formation and prevent spots and freckles", is made within the scope of the laws of each country to announce effective usage of the cosmetic to users.

The cosmetic of the present invention preferably contains at least one polyhydric alcohol selected from 1,3-butanediol, dipropylene glycol, isoprene glycol, 1,2-pentanediol, 2,4-hexylene glycol, 1,2-hexanediol, and 1, 2-octanediol in addition to a compound represented by the general formula (I). The polyhydric alcohols have an action of enhancing the skin-whitening effect of the cosmetic of the present invention.
Although the content of each of the polyhydric alcohols is not particularly limited, the content is preferably 0.1 to 30% by mass, more preferably 1 to 20% by mass, and further preferably 5 to 10% by mass. Also, the content may also be preferably 5- to 50-fold by mass and further preferably 5- to 20-fold by mass based on the compound represented by the general formula (I).

Besides, the cosmetic of the present invention preferably further contains a surfactant in addition to a compound represented by the general formula (I).

Examples of the surfactant include, anionic surfactants such as fatty acid soaps (such as sodium laurate and sodium palmitate), potassium laurylsulfate, and triethanolamine alkylsulfate ether; cationic surfactants such as trimethyl ammonium stearyl chloride, benzalkonium chloride, and laurylamine oxide; amphoteric surfactants such as imidazoline-based amphoteric surfactants (such as disodium 2-cocoyl-2-imidazolinium hydroxide-1-carboxyethyloxylate),betaine-basedsurfactants (such as alkyl betaine, amide betaine, and sulfo betaine), and acylmethyl taurine; nonionic surfactants such as sorbitan fatty acid esters (such as sorbitan monostearate and sorbitan sesquioleate), glycerin fatty acids (such as glyceryl monostearate), propyleneglycol fatty acid esters (such as propyleneglycol monostearate), cured castor oil derivatives, glycerin alkylether, POE sorbitan fatty acid esters (such as POE sorbitan monolaurate, POE sorbitan monooleate, and polyoxyethylene sorbitan monostearate), POE sorbit fatty acid esters (such as POE-sorbit monolaurate), POE glycerin fatty acid esters (such as POE-glyceryl monoisostearate), POE fatty acid esters (such as POE monolaurate, POE monooleate, and POE distearate), POE alkyl ethers (such as POE-cetyl ether and POE 2-octyldodecyl ether), POE alkylphenyl ethers (such as POE nonylphenylether), pluronic (registered trademark), POE/POP alkyl ethers (such as POE/POP2-decyltetradecyl ether), tetronic (registered trademark), POE castor oil/cured castor oil derivatives (such as POE castor oil and POE cured castor oil), sucrose fatty acid ester, and alkyl glycoside.
The cosmetic of the present invention preferably contains a nonionic surfactant. This is because the skin-whitening effect of the cosmetic can be further improved by using a compound represented by the general formula (I) and a nonionic surfactant in combination.
Although the content of the surfactant is not particularly limited, the content is preferably 0.1 to 10% by mass and further preferably 0.2 to 5% by mass. Also, the content is preferably 0.1-to 20-fold by mass and further preferably 0.2- to 20-fold by mass based on a compound represented by the general formula (I).

The cosmetic of the present invention also preferably further contains a UV protector. The UV protector to be contained in a cosmetic or quasi-drug of the present invention is not particularly limited as long as the UV protector has an ability to reduce exposure of ultraviolet light, which is a complicating factor of spots or the like, to the skin. Examples of the UV protector include various fine particles having ultraviolet light scattering effect and compounds having ultraviolet light absorbing effect.

As the fine particles, mica, talc, kaolin, synthetic mica, calcium carbonate, magnesium carbonate, silicic anhydride (silica), aluminum oxide, and barium sulfate can be given, and as the organic fine particles, polyethylene powder, polymethyl methacrylate, nylon powder, and organopolysiloxane elastomer are preferably given.

In general, such compounds having ultraviolet light absorbing effect are not particularly limited as long as the compounds have aromatic rings. Examples of the compounds include: vitamin Bs such as vitamin B₁, vitamin B₂, vitamin B₃, vitamin B₆, and vitamin B₁₂; and aromatic ring/heterocyclic vitamins such as vitamin Es, ubiquinones, and folates. Examples thereof further include para-aminobenzoic acid-based ultraviolet light absorbers, anthranilic acid-based ultraviolet light absorbers, salicylic acid-based ultraviolet light absorbers, cinnamic acid-based ultraviolet light absorbers, benzophenone-based ultraviolet light absorbers, saccharide ultraviolet light absorbers, 2-(2'-hydroxy-5'-t-octylphenyl)benzotriazole, and 4-methoxy-4'-t-butyldibenzoylmethane, which are used as ultraviolet light absorbers.

The cosmetic of the present inventionmay contain various components to be generally used in drugs, cosmetics, or the like, that is, fats and oils, waxes, hydrocarbons, fatty acids, higher alcohols, esters, oil solutions, aqueous components, powder components, humectants, thickeners, colorants, flavors, antioxidants, pH-adjusters, chelating agents, antiseptics, UV protectors, vitamins, other skin-whitening agents, and anti-inflammatory agents as well as the above-described components.

Specific examples of fats and oils and waxes include: a macadamia nut oil, an avocado oil, a corn oil, an olive oil, a rapeseed oil, a sesame oil, a castor oil, a safflower oil, a cottonseed oil, a jojoba oil, a coconut oil, a palm oil, a liquid lanolin, a cured coconut oil, a cured oil, a haze wax, a cured castor oil, a beeswax, a candelilla wax, a carnauba wax, a ibota wax, a lanolin, a reduced lanolin, a hard lanolin, and a jojoba wax; hydrocarbons such as liquid paraffin, squalane, pristane, ozokerite, paraffin, ceresin, vaseline, and microcrystalline wax; higher fatty acids as fatty acids such as oleic acid, isostearic acid, lauric acid, myristic acid, palmitic acid, stearic acid, behenic acid, and undecylenic acid; higher alcohols such as cetyl alcohol, stearyl alcohol, isostearyl alcohol, behenyl alcohol, octyldodecanol, myristyl alcohol, and cetostearyl alcohol; synthetic ester oils such as esters such as cetyl isooctanoate, isopropyl myristate, hexyldecyl isostearate, diisopropyl adipate, di-2-ethylhexyl sebacate, cetyl lactate, diisostearyl malate, ethylene glycol di-2-ethyl hexanoate, neopentylglycol dicaprate, glycerol di-2-heptyl undecylate, glycerol tri-2-ethylhexanoate, trimethylolpropane tri-2-ethylhexanoate, trimethylolpropane triisostearate, pentaerythritol tetra-2-ethylhexonate, dimer dilinoil linoleic dimmer acid, dilinoleic dimmer acid (such as phytosteryl/isostearyl/cetyl/stearyl/behenyl), and N-Lauroyl glutamic acid (phytosteryl/2-octyldodecyl); silicone oils as lubricants, such as chainpolysiloxanes such as dimethylpolysiloxane, methylphenylpolysiloxane, and diphenylpolysiloxane, ring polysiloxanes such as octamethylcyclotetrasiloxane, decamethylcyclopentasiloxane, and dodecamethylcyclohexanesiloxane, and modified polysiloxanes such as amino-modified polysiloxane, polyether-modified polysiloxane, alkyl-modified polysiloxane, and fluorine-modified polysiloxane.

Examples of the humectants include polyhydric alcohols such as polyethylene glycol, glycerin, diglycerin, erythritol, sorbitol, xylitol, maltitol, and propylene glycol, sodium pyrrolidone carboxylate, lactic acid, and sodium lactate. Examples of the thickeners include guar gum, quince seed, carrageenan, galactan, arabic gum, pectin, mannan, starch, xanthan gum, curdlan, methyl cellulose, hydroxyethyl cellulose, carboxymethyl cellulose, methylhydroxypropyl cellulose, chondroitin sulfate, dermatan sulfate, glycogen, heparan sulfate, hyaluronic acid, sodium hyalurate, tragacanth gum, keratan sulfate, chondroitin, mucoitin sulfate, hydroxyethyl guar gum, carboxymethyl guar gum, dextran, keratosulfate, locust bean gum, succinoglucan, caronic acid, chitin, chitosan, carboxymethyl chitin, agar, polyvinyl alcohol, polyvinyl pyrrolidone, carboxyvinyl polymer, sodium polyacrylate, polyethylene glycol, and bentonite.

The inorganic pigments include red iron oxide, yellow iron oxide, black iron oxide, cobalt oxide, ultramarine blue, iron blue, titanium oxide, and zinc oxide, whose surfaces may be treated. The pearl agents include mica titanium, fish scale foil, and bismuth oxychloride, whose surfaces may be treated. The organic dyes include Red No. 202, Red No. 228, Red No. 226, Yellow No. 4, Blue No. 404, Yellow No. 5, Red No. 505, Red No. 230, Red No. 223, Orange No. 201, Red No. 213, Yellow No. 204, Yellow No. 203, Blue No. 1, Green No. 201, Purple No. 201, and Red No. 204, which may be laked.

As a skin-whitening component or a skin-whitening agent other than the compound represented by the general formula (I), there are given, for example, pantetheine-S-sulfonic acid, isoferulic acid, ascorbic acid-glucoside, ascorbyl phosphate, 4-(C₄₋₁₀) alkyl resorcinols such as 4-n-butyl resorcinol, arbutin, koj ic acid, linoleic acid, methyl linoleate, tranexamic acid, and methylamide tranexamate.

The cosmetic of the present invention may contain a lower alcohols (such as ethanol and isopropanol), or a compound other than the above-described vitamins, that is, vitamin A or a derivative thereof, vitamin Ds, pantothenic acid, pantethine, coenzyme Q10, etc.

Although the dosage form of the cosmetic of the present invention may be any one and is not limited as long as the dosage form can be employed in such cosmetics or quasi-drugs, the skin-whitening cosmetic or skin-whitening quasi-drug of the present invention are desirably in a form suitable for retaining a compound in the skin for a long period of time, such as a lotion, emulsion, ointment, cream, or lotion, because a compound represented by the general formula (I) permeates through the skin and exhibits its skin-whitening effect and melanin formation inhibitory action. Also, a pack is preferable because the effect caused by blocking may be provided.

The cosmetic of the present invention can be produced in the same way as a method of producing a general cosmetic except that the cosmetic contains a compound represented by the general formula (I).

Hereinafter, the present invention will be described in more detail with reference to Examples and Test Examples, but it is obvious that the present invention is not limited to the Examples and Test Examples.

### [Examples]

### <Production Example> Synthesis of 2,6-di-tert-butyl-4-(2'-thenoyl)phenol (Compound (1))

To 23.2 g of 3,5-di-tert-butyl-4-hydroxybenzoic acid was added 60 g of thionyl chloride, and the mixture was stirred for 1 hour at room temperature, followed by distillation-off of excess thionyl chloride under reduced pressure. The residue was dissolved with 300 ml of carbon disulfide, and 13.5 g of aluminum chloride was added thereto. The mixture was stirred at 40°C for 30 minutes, and 88.2 g of thiophene was added thereto. Subsequently, the reaction solution was poured into 10% hydrochloric acid, and extraction was performed with dichloromethane. The extract was dried with anhydrous sodium sulfate and concentrated, and the concentrate was purified by fractionation using a silica gel column, to thereby produce Compound (1).

### <Example 1>

In accordance with the following prescription, an oil-in-water cream was prepared. That is, the components described in (A) were mixed and heated to 80°C. On the other hand, the components described in (B) were mixed and heated to 80°C. The mixture (B) was added to the mixture (A), and the whole was emulsified by stirring and cooled to 35°C, to thereby produce cream 1.

| | | |
|---|---|---|
| (A) | POE (30) cetyl ether | 2.0% by mass |
| | Glycerine monostearate | 10.0% by mass |
| | Liquid paraffin | 10.0% by mass |
| | Vaseline | 4.0% by mass |
| | Cetanol | 5.0% by mass |
| | γ-tocopherol | 0.05% by mass |
| | Dibutylhydroxytoluene (BHT) | 0.01% by mass |
| | Butyl paraben | 0.1% by mass |
| | Compound (1) | 0.7% by mass |
| (B) | 1,3-Butanediol | 10.0% by mass |
| | Purified water | 58.14% by mass |

### <Example 2>

In accordance with the following prescription, an oil-in-water cream was prepared. That is, the components described in (A) were mixed and heated to 80°C. On the other hand, the components described in (B) were mixed and heated to 80°C. The mixture (B) was added to the mixture (A), and the whole was emulsified by stirring and cooled to 35°C, to thereby produce cream 2.

| | | |
|---|---|---|
| (A) | POE (30) cetyl ether | 2.0% by mass |
| | Glycerine monostearate | 10.0% by mass |
| | Liquid paraffin | 10.0% by mass |
| | Vaseline | 4.0% by mass |
| | Cetanol | 5.0% by mass |
| | γ-tocopherol | 0.05% by mass |
| | BHT | 0.01% by mass |
| | Butyl paraben | 0.1% by mass |
| | Compound (1) | 0.05% by mass |
| (B) | 1,3-Butanediol | 10.0% by mass |
| | Purified water | 58.79% by mass |

### <Example 3>

In accordance with the following prescription, an emulsion was prepared. That is, the components described in (A) were mixed and heated to 70°C. On the other hand, the components described in (B) were mixed and heated to 70°C. The mixture (A) was added to the mixture (B) to achieve preliminary emulsification, and the whole was further emulsified using a homomixer. After completion of emulsification, the whole was cooled to 30°C while being stirred, to thereby produce an emulsion.

| | | |
|---|---|---|
| (A) | Synthetic cetaceum | 2.5% by mass |
| | Cetanol | 1.0% by mass |
| | Squalane | 4.0% by mass |
| | Stearic acid | 1.0% by mass |
| | POE(25)monostearate | 2.2% by mass |
| | Glycerin monostearate | 0.5% by mass |
| | γ-tocopherol | 0.05% by mass |
| | BHT | 0.01% by mass |
| | Butyl paraben | 0.1% by mass |
| | Compound (1) | 1.0% by mass |
| (B) | 1,3-butandiol | 3.0% by mass |
| | Propylene glycol | 7.0% by mass |
| | Xanthan gum | 0.1% by mass |
| | Carboxy vinyl polymer | 0.2% by mass |
| | Potassium hydroxide | 0.2% by mass |
| | Purified water | 77.14% by mass |

### <Example 4>

In accordance with the following prescription, a lotion was prepared. The components described in (A) were mixed and dissolved at room temperature. On the other hand, the components described in (B) were mixed and dissolved at room temperature. The mixture (A) was added to the mixture (B) to achieve solubilization, to thereby produce a lotion.

| | | |
|---|---|---|
| (A) | POE(20)sorbitan monolaurate | 1.5% by mass |
| | POE(20)monolaurate | 0.5% by mass |
| | Ethanol | 10.0% by mass |
| | γ-tocopherol | 0.02% by mass |
| | Compound (1) | 0.01% by mass |
| (B) | Glycerin | 5.0% by mass |
| | Propylene glycol | 4.0% by mass |
| | Sodium isoferulate | 0.05% by mass |
| | Citric acid | 0.15% by mass |
| | Sodium citrate | 0.1% by mass |
| | Purified water | 78.67% by mass |

### <Example 5>

In accordance with the following prescription, a pack was prepared. The components described in (A) were dispersed and dissolved at room temperature, and the mixture (B) was added to and dissolved in the mixture (A) uniformly, to thereby produce a pack.

| | | |
|---|---|---|
| (A) | Polyvinyl alcohol | 15.0% by mass |
| | Purified water | 40.0% by mass |
| (B) | Bisabolol | 0.5% by mass |
| | γ-tocopherol | 0.02% by mass |
| | Ethanol | 4.0% by mass |
| | Compound (1) | 2.0% by mass |
| | POE(8)·POP(55) copolymer | 3.0% by mass |
| | Purified water | 35.48% by mass |

### <Test Example 1> Melanin formation inhibition test of Compound (1)

The melanin formation inhibitory action of Compound (1) was evaluated using thiouracil (in this test, thiouracil labeled with ¹⁴C was used), which is specifically taken into cells in a melanin synthesis process. A complete medium for culturing melanocytes (from Kurabo Industries Ltd.) was added to 15 wells of a 24-well plate in an amount of 2 ml per well, and human normal melanocytes (from Kurabo Industries Ltd.) were inoculated into each well at a concentration of 1.5 × 10⁴ cells/cm². The cells were cultured at 37°C for 24 hours under 5% carbon dioxide atmosphere. Thereafter, in all the wells, the medium was exchanged under the following conditions. That is, a fresh complete medium for culturing melanocytes was added to three wells (control), complete mediums for culturing melanocytes containing Compound (1) at concentrations of 1.0 × 10⁻³ mM, 2.0 × 10⁻³ mM, and 4.0 × 10⁻³ mM were added to three wells each (total: nine wells), and a complete medium for culturing melanocytes containing 0.1 mM phenylthiourea, which is known as a tyrosinase inhibitor, was added to other three wells. In addition, to those 15 wells was added ¹⁴C-thiouracil (thiouracil labeled with ¹⁴C) was added at 0.25 × 10⁻⁶ Ci (curie). The cells were cultured under the same conditions as above for further three days. After completion of the culture, the culture solution was removed from the wells, and the cells were washed with PBS (phosphate buffered saline) and detached with a medium containing trypsin and EDTA from the bottom of each well to produce cell suspensions, followed by centrifugation to collect the cells. The numbers of the cells were counted using a blood cell counting chamber. Thereafter, the ¹⁴C-thiouracil level in the cells collected from each well was determined using a liquid scintillation counter. The percentage of a radiation dose of the cells cultured in each medium containing a test substance based on a radiation dose of the cells collected from the control wells was calculated to determine a melanin level (%). That is, it is considered that the smaller the radiation dose taken in the cells is, the smaller the melanin level is, resulting in a large melanin inhibition titer. The results are shown in Table 1.

**Table 1**

| Component added to medium | Concentration (mM) | Melanin level (%) |
|---|---|---|
| - (Control) | - | 100±0.6 |
| Compound (1) | 1.0×10⁻³ | 48.5±2.3 |
| | 2.0×10⁻³ | 27.9±0.8 |
| | 4.0×10⁻³ | 17.0±0.8 |
| Phenylthiourea | 0.1 | 20.3±3.8 |

The results of Table 1 reveal that Compound (1) contained in the cosmetic of the present invention has a concentration-dependent melanin formation inhibitory action. Phenylthiourea (0.1 mM) and Compound (1) (4.0 × 10⁻³ mM) were found to have almost the same melanin formation inhibition titers. The results reveal that Compound (1) has a melanin formation inhibition titer that is 20 times or more higher than that of phenylthiourea (positive control). Although phenylthiourea is not practical because of its toxicity, phenylthiourea is generally used as a positive control in such an experiment because phenylthiourea is a substance having a very strong melanin formation inhibitory action compared with a melanin formation inhibitor that is now practically used. The concentration of Compound (1) in the composition, 4 × 10⁻³ mM, corresponds to about 0.0001% by mass.

### <Test Example 2> Use test 1 of cosmetic of the present invention

Subsequently, based on the above-described results, the effects of the cosmetics of the present invention on prevention/amelioration of melanopathy were examined. Use tests of creams 1 and 2 produced in Examples 1 and 2 were performed to examine the melanin formation inhibitory actions on ultraviolet irradiation. On the other hand, a cream prepared in the same way as Example 1 except that purified water was used instead of Compound (1) (control) was used as a comparative example.

Three sites with a size of 1.5 cm × 1.5 cm were specified on the medial side of the forearm of each of ten subjects, and cream 1 produced in Example 1 (containing 0.7% Compound (1)), cream 2 produced in Example 2 (containing 0.05% Compound (1)), and a control cream as a comparative example were separately applied to one of the three sites, another site, and the other site, respectively, three times a day for one week. One week later, the specified sites were irradiated with ultraviolet light continuously for three days while protecting the area other than the three specified sites from ultraviolet irradiation. The amount of the energy of ultraviolet irradiation was found to be equivalent to 1.0 MED (Minimum Erythema Dose) of each subject (equivalent to 3.0 MED in total), which was preliminarily determined. The creams were applied three times a day continuously for further 21 days including the initial three days of ultraviolet irradiation. At 7 and 21 days after the initial ultraviolet irradiation, three professional evaluators evaluated degrees of pigmentation on the sites irradiated with ultraviolet light according to the following criteria. Based on the evaluation results, the averages of scores given by the evaluators were calculated for the respective specified sites. The results are shown in Table 2.

### Pigmentation degree score

| Score: | Note |
|---|---|
| 0: | Site with no change |
| 0.5: | Slightly blackened site with poorly-defined border |
| 1.0: | Blackened site with clearly-defined border |
| 1.5: | Moderately blackened site with clearly-defined border |
| 2.0: | Severely blackened site with clearly-defined border |

| | |
|---|---|
| * Middle scores between the scores are also employed. | |

**Table 2**

| | Control | Cream 2 containing 0.05% Compound (1) | Cream 1 containing 0.7% Compound (1) |
|---|---|---|---|
| 7 days later | 1.18±0.45 | 1.07±0.42 | 1.10±0.45 |
| 21 days later | 0.92±0.34 | 0.78±0.29 | 0.75±0.32 |

As shown in Table 2, at 7 days after ultraviolet irradiation, there were no obvious differences and no significant differences (Paired-T-test) among the scores of the control, cream 2 (containing 0.05% Compound (1)), and cream 1 (containing 0.7% Compound (1)). That is, it is consideredthatpigmentationwas caused at similar degrees on the sites irradiated with ultraviolet light. However, at 21 days after the initial ultraviolet irradiation, there were significant differences (P < 0.05, each comparison) between the scores of the control and cream 2 and between the scores of the control and cream 1. Although there was no significant difference between the scores of cream 2 and cream 1, cream 1 is considered to have an excellent action of suppressing pigmentation because the score of cream 1 was smaller than that of cream 2.

Meanwhile, at 7 and 21 days after the initial ultraviolet irradiation, lightness values (L values) of the three specified sites and adjacent sites thereof (protected sites) were measured using a colorimeter (Konica Minolta Colorimeter CR300). Based on the measurement results, averages of the L values were calculated for the respective specified sites of the ten subjects. Moreover, differences between the L values of the specified sites (ultraviolet-irradiated sites) at 21 days after the initial ultraviolet irradiation and the L values of the adjacent sites (ultraviolet-unirradiated) (ΔL value: L value of adjacent site - L value of ultraviolet-irradiated site) were calculated. The results are shown in Table 3.

**Table 3**

| | Control | Cream 2 containing 0.05% Compound (1) | Cream 1 containing 0.7% Compound (1) | Unirradiated |
|---|---|---|---|---|
| L value at 7 days after | 61.03 | 60.85 | 60.67 | 66.42 |
| ultraviolet irradiation | ±4.49 | ±3.87 | ±4.00 | ±1.90 |
| L value at 21 days after | 62.94 | 63.60 | 63.66 | 66.67 |
| ultraviolet irradiation | ±3.25 | ±3.25 | ±3.23 | ±3.21 |
| ΔL value at 21 days after | 3.73 | 3.07 | 3.01 | --- |
| ultraviolet irradiation | ±1.43 | ±1.42 | ±1.49 | --- |

As shown in Table 3, at day 7 after the ultraviolet irradiation, the L values (lightness values) of the cream 1 (containing 0.7% Compound (1))-applied sites, the cream 2 (containing 0.05% Compound (1))-applied sites, and control-applied sites were found to be lower than the L value of the ultraviolet-unirradiated site. That is, it was found that pigmentation caused by ultraviolet irradiation reduced the lightness value of the skin. Moreover, comparison of ΔL values, showing differences between L values (lightness values) of the respective applied sites and L value of the unirradiated sites at 21 days after initial ultraviolet irradiation, revealed that the ΔL values (showing differences from the L value of the unirradiated sites) of the sites applied with cream 1 and cream 2 were smaller than those of the sites applied with control, and the lightness values at the sites applied with cream 1 and cream 2 recovered more. That is, cream 1 and cream 2 were found to have actions of ameliorating pigmentation. Paired-T-test for the sites applied with cream 1 and cream 2 based on the sites applied with control was performed, and the results revealed that there were significant differences (P <0.05).

### <Test Example 3> Use test 2 of cosmetic of the present invention

The prescription of Example 1 was performed except that 1,2-pentanediol, glycerin, and water were used instead of 1,3-butanediol, to thereby produce cream 3, cream 4, and cream 5, respectively. Cream 1 produced in Example 1, cream 3, cream 4, and cream 5 were used to perform ultraviolet irradiation and sample application on four sites with a size of 1.5 cm × 1.5 cm, specified on the medial side of the forearm of each of subjects, by almost the same method as that described in Test Example 2, and at 7 and 21 days after the ultraviolet irradiation, lightness values (L values) were measured by a colorimeter, followed by calculation of differences between the resultant L values and L value of unirradiated sites. The results are shown in Table 4.

**Table 4**

| | Cream 1 | Cream 3 | Cream 4 | Cream 5 | Unirradiated |
|---|---|---|---|---|---|
| L value at 7 days after | 60.67 | 60.85 | 60.76 | 60.72 | 66.42 |
| ultraviolet irradiation | ±4.00 | ±3.87 | ±3.90 | ±4.02 | ±1.90 |
| L value at 21 days after | 63.66 | 63.65 | 63.28 | 63.26 | 66.71 |
| ultraviolet irradiation | ±3.23 | ±3.21 | ±3.31 | ±3.28 | ±3.24 |
| ΔL value at 21 days after | 3.05 | 3.06 | 3.43 | 3.45 | --- |
| ultraviolet irradiation | ±1.49 | ±1.42 | ±1.51 | ±1.61 | --- |

As shown in Table 4, the evaluation using a colorimeter revealed that, at 7 days after the ultraviolet irradiation, the L values (lightness values) of the sites applied with cream 1, cream 3, cream 4, and cream 5 were lower than the L value of the ultraviolet-unirradiated site. That is, it was found that pigmentation caused by ultraviolet irradiation reduced the lightness value of the skin. Moreover, comparison of ΔL values, showing differences between L values (lightness values) of the respective applied sites and L value of the unirradiated sites at 21 days after the ultraviolet irradiation, revealed that that the ΔL values of the sites applied with cream 1 containing 1, 3-butanediol and cream 3 containing 1,2-pentanediol were smaller than those of the sites applied with cream 5 containing water instead of those compounds, and the creams 1 and 3 promoted recovery of the lightness values. Meanwhile, cream 4 containing glycerin instead of 1,3-butanediol was found to have a little lower effect of promoting recovery of lightness values compared with cream 1 and cream 2. That is, cream 1 and cream 3, which contain divalent polyhydric alcohols such as 1,3-butanediol and 1,2-pentanediol, are considered to have an excellent action of preventing/ameliorating pigmentation.

The cosmetics or quasi drugs of the present invention were also confirmed to be highly safe because all of creams 1 to 5 of the present invention caused no unfavorable reactions on the applied sites.

### <Test Example 4> Normal human melanocytes tyrosinase activity inhibition test

Human normal melanocytes (from Kurabo Industries Ltd.), cultured in a medium for culturing melanocytes (Medium 254, from Kurabo Industries Ltd.), were collected using a cell scraper, and extraction was performed for the cells using PreteoExtract^{™} Subcellular Proteome Extraction Kit(Calbiochem), to thereby produce a protein solution, which was used as a crude tyrosinase protein solution. The dopa-oxidizing activity of the tyrosinase protein solution was measured to evaluate the tyrosinase activity-inhibiting action of Compound (1).
A 0.1% dopa solution, serving as a substrate, was prepared by dissolving L-β-(3,4-dihydroxyphenyl)alanine (L-DOPA) in 10 mM sodium phosphate buffer (pH 6.8) at a concentration of 0.1 (w/v)%. A crude tyrosinase protein solution, prepared to 1 to 5 µg/80 µL, was fed into a 96-well plate in an amount of 80 µL per well, and solutions of 10 µM, 100 µM, and 1,000 µM Compound (1) in DMSO were separately added to three wells each in an amount of 20 µL per well. Meanwhile, as positive controls, solutions of 10 µM, 100 µM, and 1,000 µM phenylthiourea, known as a tyrosinase activity inhibitor, in DMSO were separately added in the same way as above to different three wells each in an amount of 20 µL per well. In addition, as a control, DMSO was added to another different three wells each in an amount of 20 µL per well. Immediately after 100 µL each of the 0.1% dopa solution was added to the wells each containing the above-described test solution, absorbances at 450 nm were measured using a plate reader and were defined as 0-minute values. The 96-well plate was incubated at 37°C for 10 minutes in the plate reader, and the absorbances at 450 nm were measured again and defined as 10-mintue values. A value calculated by subtracting the 0-mintue value from the 10-mintue value of each well was defined as a 0→10 minute absorbance change value, and the average of 0→10 minute absorbance change value of control was defined as 100%, followed by measurement of dopa-oxidizing activities in the cases of treatments with various concentrations of test substances. The results are shown in Table 5.

**Table 5**

| | Concentration of added substance | Dopa-oxidizing activity |
|---|---|---|
| Control | 0 | 100.00±4.59 |
| Compound (1) | 1 µM | 103.55±1.34 |
| | 10 µM | 102.52±3.68 |
| | 100 µM | 102.52±2.19 |
| Phenylthiourea (Positive control) | 1 µM | 64.07±1.53 |
| | 10 µM | 22.52±1.09 |
| | 100 µM | 4.73±2.07 |

In the cases where Compound (1) was added, the dopa-oxidizing activities were almost the same as the activity of control regardless of the concentrations of Compound (1) added. That is, it was found that Compound (1) did not inhibit the activity of tyrosinase extracted from normal human melanocytes. The concentration of Compound (1) added, 100 µM, is 25-fold higher than the concentration of Compound (1) that inhibited melanin formation by 80% or more in the cultured cell system shown in Test Example 1. On the other hand, the higher the concentration of phenylthiourea added is, the lower the dopa-oxidizing activity is, which revealed that phenylthiourea had an action of inhibiting the activity of tyrosinase. This suggests that Compound (1) inhibits melanin formation by a mechanism entirely different from that of direct inhibition to tyrosinase, which is different from a common melanin formation inhibitor. Such a mechanism is considered to include inhibition of expression of tyrosinase and tyrosinase-related proteins such as Trp-1 and Trp-2, indirect inhibition of the tyrosinase activity by metabolism products in cells, and inhibition of transport of melanin produced.

### <Test Example 5> Determination of expression of tyrosinase and tyrosinase-related protein (Trp-1, Trp-2)

A cell suspension of human normal melanocytes (from Kurabo Industries Ltd.) in a medium for culturing melanocytes (Medium 254, from Kurabo Industries Ltd.) was prepared at 7 × 10⁴ cells/mL and inoculated into ϕ10-cm culture petri dishes in an amount of 10 mL per dish, and the cells were cultured in a CO₂ incubator (37°C, 5% CO₂). The next day, the medium was removed, and a medium for culturing melanocytes containing 2 µM or 5 µM Compound (1) or a medium for culturing melanocytes (control) was added in an amount of 10 mL per dish, followed by culture in a CO₂ incubator for 72 hours. After 72-hour culture, the medium was removed, and the cells were washed with phosphate buffered saline (PBS) and collected using a cell scraper. A protein extraction solution (0.1% NP-40, 0.01% SDS, 10 mM sodium phosphate buffer (pH 6.8), protease inhibitor) was added to the cell pellets to suspend the cells, and the suspension was shaken at 4°C for 1 hour. The resultant was centrifuged at 4°C and 15,000 rpm for 10 minutes, and the supernatant was collected and used as a crude protein extract.
The crude protein extract (5 µg) was subjected to SDS-polyacrylamide electrophoresis in accordance with a conventional method, and electrophoresed proteins were transferred to a PVDF membrane, which was immersed in TPBS (PBS containing 0.1% (w/v) tween-20) containing 3% skimmed milk for 1 hour (room temperature). The membrane was washed with TPBS and allowed to react with TPBS containing a primary antibody capable of recognizing tyrosinase or a tyrosinase-related protein for 1 hour (room temperature). The antibodies used and dilution ratios are as follows.
Tyrosinase; anti-tyrosinase (H-109) polyclonal antibody (rabbit), (from Santa Cruz Biotechnology, Inc., SC-15341), diluted to 1/400
Trp-1; anti-Trp-1 (H-90) polyclonal antibody (rabbit), (from Santa Cruz Biotechnology, Inc., SC-25543), diluted to 1/400
Trp-2; anti-Trp-2 (D-18) polyclonal antibody (goat), (from Santa Cruz Biotechnology, Inc., SC-10451), diluted to 1/250

The membrane was washed with TPBS and allowed to react with TPBS containing a secondary antibody for 1 hour (room temperature). The antibodies used and dilution ratios are as follows.
Tyrosinase and Trp-1; ECL Anti-rabbit IgG, Horseradish peroxidase-linked species-specific whole antibody (donkey), from Amersham Biosciences, NA934), diluted to 1/15,000
Trp-2; Anti-goat IgG, Horseradish peroxidase-conjugated antibody (donkey), (from Santa Cruz Biotechnology, Inc., SC-2033), diluted to 1/7,500
The membrane was washed with TPBS, and proteins were detected using an ECL Plus western blotting detection system. Detection was performed using a cooled CCD camera-equipped chemiluminescence detection system (from ATTO Corporation). The results are shown in Fig. 1.

Compound (1) did not affect the expression levels of tyrosinase and tyrosinase-related proteins (Trp-1, Trp-2) even when the concentration of Compound (1) was 5 µM, which was a concentration higher than the concentration at which the melanin formation inhibitory action was confirmed in <Test Example 1>. The results further endorsed that the melanin formation inhibitory action was provided by a novel mechanism.

### [Industrial Applicability]

The present invention provides a skin-whitening cosmetic which is highly effective for prevention/amelioration of melanopathy such as wrinkles/freckles. Moreover, the cosmetic of the present invention can be used safely.
The present invention can be applied to a skin-whitening cosmetic or a skin-whitening quasi-drug.

## Claims

1. A skin-whitening cosmetic comprising a compound represented by the following general formula (I) and/or a salt thereof: 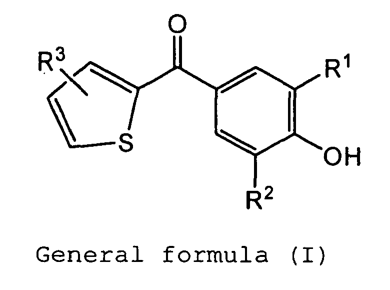 wherein, R¹ and R² independently represent a secondary or tertiary alkyl group having 3 to 7 carbon atoms, and R³ represents a hydrogen atom, an alkyl group having 1 to 4 carbon atoms, or an alkoxy group having 1 to 4 carbon atoms.

2. A skin-whitening cosmetic according to Claim 1, wherein the compound represented by the general formula (I) is a compound represented by the following general formula (II): 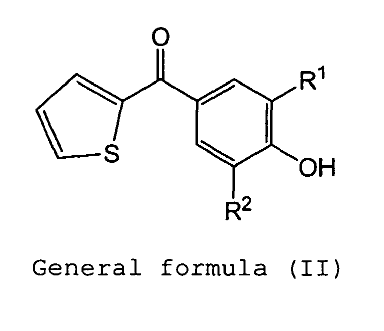 wherein, R¹ and R² independently represent a secondary or tertiary alkyl group having 3 to 7 carbon atoms.

3. A skin-whitening cosmetic according to Claim 2, wherein the compound represented by the general formula (II) is 2,6-di-tert-butyl-4-(2'-thenoyl)phenol (Compound (1)).

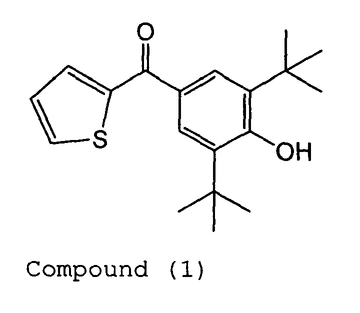

4. A skin-whitening cosmetic according to any one of Claims 1 to 3, wherein the skin-whitening cosmetic is used for prevention/amelioration of melanopathy.

5. A skin-whitening cosmetic according to any one of Claims 1 to 4, wherein a content of the compound represented by the general formula (I) is 0.0001 to 3% by mass.

6. A skin-whitening cosmetic according to any one of Claims 1 to 5, further comprising at least one polyhydric alcohol selected from 1,3-butanediol, dipropylene glycol, isoprene glycol, 1,2-pentanediol, 2,4-hexylene glycol, 1,2-hexanediol, and 1,2-octanediol.
